# FASCICULE DE BREVET EUROPEEN

(11) **EP 4 090 224 B1**
(45) Date de publication et mention de la délivrance du brevet: **26.03.2025**
(21) Numéro de dépôt: 21719948.8
(22) Date de dépôt: 15.01.2021
(51) Int. Cl.: A61B 1/005, A61B 1/00, A61B 1/008, A61B 1/01

(54) **STRUCTURE DE FLEXION DÉCOUPÉE POUR DISPOSITIF MÉDICAL**
AUSGESCHNITTENE BIEGESTRUKTUR FÜR MEDIZINISCHES GERÄT
CUT-OUT BENDING STRUCTURE FOR MEDICAL DEVICE

(30) Priorité: 17.01.2020 FR 2000478
(43) Date de publication de la demande: 23.11.2022
(73) Titulaire: Axess Vision Technology, 37300 Joue les Tours (FR)
(72) Inventeur: HALLAUER, Emmanuel, 37190 SACHE (FR); COCHARD, Pascal, 37150 DIERRE (FR)
(74) Mandataire: Cabinet Beau de Loménie
(86) Numéro de dépôt international: PCT/FR2021/050071
(87) Numéro de publication internationale: WO 2021/144542

(56) Documents cités:
- EP-A1- 2 130 478
- FR-A1- 2 740 688
- JP-A- 2013 223 655
- US-A1- 2015 087 910

## Description

### Domaine Technique

La présente invention concerne le domaine technique des dispositifs médicaux au sens général permettant d'accéder à l'intérieur d'un corps comme une cavité ou un canal par exemple et elle vise plus précisément les dispositifs médicaux du type cathéter et de préférence, les dispositifs médicaux du type endoscope.

L'objet de l'invention trouve une application particulièrement avantageuse pour les endoscopes à caractère réutilisable ou à usage unique.

La présente invention concerne plus précisément la structure de flexion permettant d'orienter la tête distale de tels dispositifs médicaux du type cathéter ou endoscope, cette tête distale étant adaptée pour assurer de multiples fonctions telles que la visualisation, l'amenée de fluide, l'aspiration de fluide, l'amenée d'instruments, la réalisation de prélèvements ou d'opérations de chirurgie par exemple.

Le dispositif médical du type cathéter ou endoscope équipé de la structure de flexion de la tête distale conforme à l'invention trouve des applications particulièrement avantageuses pour permettre d'accéder à la surface interne d'un organe creux, d'une cavité ou d'un conduit naturel ou artificiel du corps humain en vue d'effectuer diverses opérations à des fins thérapeutiques, chirurgicales ou de diagnostic.

Le dispositif médical du type cathéter ou endoscope selon l'invention est utilisé à des fins de diagnostic, thérapeutiques ou de chirurgie pour l'inspection de toutes les parties internes du corps humain accessibles par les voies naturelles ou artificielles. Par exemple, le dispositif médical du type cathéter ou endoscope selon l'invention peut être utilisé dans le domaine des voies urinaires, des voies gastro-intestinales, du système respiratoire, du système cardiovasculaire, de la trachée, de la cavité du sinus, du système de reproduction de la femme, de la cavité abdominale ou de tout autre partie du corps humain à explorer par une voie naturelle ou artificielle.

### Technique antérieure

D'une manière générale, un endoscope médical comporte, comme décrit par exemple par la demande de brevet WO 2014/106510, une poignée de commande à laquelle est fixé un tube d'insertion. Ce tube comporte une tête distale équipée d'un système de visualisation optique permettant d'éclairer et d'examiner l'organe, la cavité ou le conduit du corps humain. En amont de cette tête distale, le tube d'insertion comporte une structure de flexion ou partie de béquillage formée de vertèbres articulées permettant l'orientation de la tête distale à l'aide d'un ou plusieurs câbles d'actionnement montés à l'intérieur du tube d'insertion. Chaque câble d'actionnement comporte une première extrémité fixée à la tête distale et une deuxième extrémité sur laquelle agit un mécanisme de commande équipant la poignée pour assurer le coulissement des câbles et par suite, le pliage de cette partie de béquillage afin d'orienter la tête distale.

La fabrication de cette partie de béquillage nécessite l'assemblage des vertèbres conduisant à une difficulté de montage et à un coût de fabrication élevé. Cette difficulté de fabrication apparaît d'autant plus que dans de nombreuses applications, il apparaît le besoin de miniaturiser un tel endoscope pour permettre son passage dans une voie d'accès à diamètre réduit. Cette miniaturisation doit tenir compte de la nécessité de monter à l'intérieur du tube d'insertion, divers appareillages adaptés pour permettre de réaliser différentes fonctions telles que l'amenée de fluide, l'aspiration de fluide, l'amenée d'instruments, effectuer des prélèvements ou des opérations de chirurgie, le passage pour les connexions du système de vision.

Le brevet US 5 002 041 décrit un endoscope dont la partie de béquillage comporte deux ressorts imbriqués pour obtenir un décalage entre les spires des ressorts afin de ménager un espace pour le montage d'un câble qui se trouve ainsi guidé en translation. Une telle solution conduit à une partie de béquillage relativement encombrante ne permettant pas de réduire la section transversale de l'endoscope sans risque d'altérer le fonctionnement des appareillages positionnés à l'intérieur du tube d'insertion.

La demande de brevet WO 2014/061842 décrit un endoscope comportant une partie de béquillage formée notamment par un ressort hélicoïdal dont des spires sont pourvues de déformations concaves alignées pour servir de guidage à un câble d'actionnement. La réalisation de ces déformations concaves sur un ressort conduit à une difficulté de fabrication et à un coût de fabrication élevé notamment pour des tubes d'insertion de faibles diamètres.

Dans l'état de la technique, il est également connu notamment par les documents EP 1 604 607, CN 107951456, CN 104605805, CN 107520273 et EP 3 195 784 de réaliser la partie de béquillage ou la structure de flexion, à l'aide d'un tube découpé par des traits de découpe d'un faisceau d'énergie pour réaliser des vertèbres tubulaires imbriquées les unes dans les autres par des zones découpées formant des pivots de rotation selon des axes de pivotement. Entre les vertèbres tubulaires sont réalisées des entailles découpées dans le tube pour former des zones de flexion pour les vertèbres tubulaires afin d'obtenir la flexion de la structure de flexion dans au moins un plan de flexion perpendiculaire aux axes de pivotement. Une telle structure de flexion présente un faible coût de fabrication tout en étant apte à pouvoir présenter une section transversale réduite.

Le document EP2130478A1 divulgue une structure de flexion pour un tube d'insertion d'un dispositif médical, comportant des vertèbres tubulaires avec une vertèbre tubulaire proximale et une vertèbre tubulaire distale et au moins un câble d'actionnement entouré par une gaine sur au moins une partie de sa longueur, la structure de flexion comportant un tube découpé par des traits de découpe pour réaliser des vertèbres tubulaires imbriquées les unes dans les autres par des zones découpées formant des pivots de rotation selon des axes de pivotement, des traits de découpe étant aménagées pour délimiter entre les vertèbres tubulaires des entailles découpées dans le tube pour former des zones de flexion pour les vertèbres tubulaires afin d'obtenir la flexion de la structure de flexion dans au moins un plan de flexion perpendiculaire aux axes de pivotement, la vertèbre tubulaire proximale comportant pour un câble d'actionnement, un système de blocage axial et radial pour la gaine comportant une découpe allongée aménagée pour délimiter deux bords d'arrêt limitant l'engagement radial de la gaine à l'intérieur de la découpe.

Toutefois, il apparaît une difficulté pour assurer le montage des câbles d'actionnement dans une position adaptée pour offrir une capacité de béquillage à la tête distale selon une grande plage angulaire. En effet, il est à noter que le câble d'actionnement coulisse à l'intérieur d'une gaine de protection fixe lors des opérations de béquillage. Il s'ensuit que le positionnement du câble d'actionnement doit être tel que d'une part, son mouvement de coulissement puisse entrainer le béquillage de la structure de flexion sur toute la plage angulaire souhaitée et d'autre part, les frottements avec la gaine doivent être limités.

De manière complémentaire, il apparaît une difficulté pour assembler une telle structure de flexion avec le tube d'insertion d'un côté et la tête distale de l'autre côté. En effet, un tel assemblage doit être sûr et efficace tout en présentant un encombrement limité.

### Exposé de l'invention

La présente invention vise donc à remédier aux inconvénients de l'état de la technique en proposant une structure de flexion pour dispositif médical du type cathéter ou endoscope, présentant un faible coût de fabrication, en étant apte à pouvoir présenter une section transversale réduite pour son passage dans une voie d'accès de faible diamètre tout en offrant une capacité suffisante de béquillage à la tête distale sans altérer les différents appareillages mis en œuvre par cet endoscope.

Pour atteindre un tel objectif, la structure de flexion pour un tube d'insertion d'un dispositif médical, comporte des vertèbres tubulaires avec une vertèbre tubulaire proximale et une vertèbre tubulaire distale et au moins un câble d'actionnement entouré par une gaine sur au moins une partie de sa longueur, la structure de flexion comportant un tube découpé par des traits de découpe d'un faisceau d'énergie pour réaliser des vertèbres tubulaires imbriquées les unes dans les autres par des zones découpées formant des pivots de rotation selon des axes de pivotement, des traits de découpe étant aménagées pour délimiter entre les vertèbres tubulaires des entailles découpées dans le tube pour former des zones de flexion pour les vertèbres tubulaires afin d'obtenir la flexion de la structure de flexion dans au moins un plan de flexion perpendiculaire aux axes de pivotement, la vertèbre tubulaire proximale comportant pour chaque câble d'actionnement, un système de blocage axial et radial pour la gaine comportant une découpe allongée aménagée pour délimiter deux bords d'arrêt limitant l'engagement radial de la gaine à l'intérieur de la découpe, cette découpe débouchant à sa partie distale dans une lumière délimitée par au moins deux lignes de découpe entre lesquelles est attachée une languette d'appui aménagée pour solliciter radialement la gaine en appui contre les deux bords d'arrêt de la découpe, cette lumière étant bordée par un rebord d'arrêt axial de la gaine sur lequel l'extrémité distale de la gaine est en appui.

Avantageusement, la vertèbre tubulaire proximale comporte à sa partie proximale, des languettes de liaison s'étendant axialement et comportant une déformation radiale pour s'engager dans la partie distale du tube d'insertion.

Selon un mode de réalisation, la vertèbre tubulaire proximale comporte entre deux languettes voisines, des dents comportant des bords d'extrémité venant en butée sur le bord de l'extrémité distale du tube d'insertion.

Selon une variante de réalisation, la vertèbre tubulaire distale est pourvue d'un système de positionnement du câble d'actionnement pour son soudage sur la vertèbre tubulaire distale, ce système de positionnement comportant une languette d'appui découpée du tube par deux lignes de découpe parallèles, en restant attachée au tube à ses extrémités par des zones d'attache et deux contre languettes s'étendant de part et d'autre de la languette d'appui en étant chacune découpée du tube par deux lignes de découpe parallèles, en restant attachée au tube à ses extrémités par des zones d'attache, le câble d'actionnement étant positionné avant soudure entre les contre languettes et la languette d'appui, au moins une soudure permettant de fusionner le câble d'actionnement à la languette d'appui.

Avantageusement, la vertèbre tubulaire distale est pourvue d'un système d'assemblage avec une tête distale comportant au moins deux découpes d'insertion pour un doigt de la tête distale, aménagées en s'ouvrant à la partie distale par un canal d'engagement pour le doigt, au moins une découpe présentant deux pointes de blocage pour un doigt de la tête distale, s'étendant de part et d'autre du canal d'engagement de cette découpe.

Selon un premier mode de réalisation, le tube est découpé par une première série de zones découpées formant des pivots de rotation et par une deuxième série de zones découpées formant des pivots de rotation s'étendant diamétralement opposée par rapport à la première série pour former des premiers axes de pivotements, des traits de découpe étant aménagées pour délimiter entre les vertèbres tubulaires, au moins une première série d'entailles pour former des zones de flexion pour les vertèbres tubulaires afin d'obtenir la flexion de la structure de flexion dans un sens de la direction perpendiculaire au premier axe de pivotement.

Conformément à l'invention, les zones découpées formant des pivots de rotation coopèrent entre elles par leur épaisseur.

Selon une variante préférée de réalisation, des vertèbres tubulaires comportent au moins une languette découpée du tube par deux lignes de découpe parallèles, en restant attachée au tube à ses extrémités par des zones d'attache, la languette étant repoussée à l'intérieur du tube pour constituer un œil de guidage pour le câble d'actionnement.

Un autre objet de l'invention est de proposer un tube d'insertion d'un dispositif médical comportant une structure de flexion conforme à l'invention, fixée à son extrémité proximale au tube et à son extrémité distale, à une tête distale.

Un autre objet de l'invention est de proposer une poignée de commande pour un dispositif médical, équipée à sa partie distale d'un tube d'insertion conforme à l'invention.

Diverses autres caractéristiques ressortent de la description faite ci-dessous en référence aux dessins annexés qui montrent, à titre d'exemples non limitatifs, des formes de réalisation de l'objet de l'invention.

### Brève description des dessins

[Fig. 1] La Figure 1 est une vue générale schématique d'un dispositif médical du type cathéter ou endoscope au sens général équipé d'une structure de flexion conforme à l'invention.
[Fig. 2] La Figure 2 est une vue de côté montrant plus particulièrement un exemple préféré de réalisation d'une structure de flexion conforme à l'invention.
[Fig. 3]La Figure 3 est une vue de côté de la structure de flexion conforme à l'invention illustrée à la Figure 2.
[Fig. 4]La Figure 4 est une vue en coupe transversale de la structure de flexion conforme à l'invention illustrée à la Figure 3.
[Fig. 5]La Figure 5 est une perspective de la structure de flexion conforme à l'invention illustrée à la Figure 3.
[Fig. 6]La Figure 6 est une vue en perspective de l'intérieur de la structure de flexion conforme à l'invention illustrée à la Figure 5.
[Fig. 7]La Figure 7 est une vue en plan montrant la structure de flexion illustrée à la Figure 5, en position fléchie ou béquillée.
[Fig. 8]La Figure 8 est une vue en perspective montrant la structure de flexion illustrée à la Figure 5, en position fléchie ou béquillée.
[Fig. 9]La Figure 9 est une vue en perspective d'une autre variante de réalisation de la structure de flexion conforme à l'invention.
[Fig. 10]La Figure 10 est une vue analogue de la structure de flexion illustrée à la Fig. 9, montrée dans une position béquillée décalée de 90°.
[Fig. 11]La Figure 11 est une vue en plan montrant la tête distale et la structure de flexion avant leur assemblage.
[Fig. 12]La Figure 12 est une vue en perspective montrant l'assemblage de la tête distale avec la structure de flexion.
[Fig. 13]La Figure 13 est une demi vue en coupe montrant le montage du câble d'actionnement pour favoriser son soudage sur la vertèbre tubulaire distale.
[Fig. 13A]La Figure 13A est une demi vue en coupe montrant un autre montage du câble d'actionnement pour favoriser son soudage sur la vertèbre tubulaire distale.
[Fig. 14]La Figure 14 est une vue en perspective montrant le tube d'insertion et la structure de flexion avant leur assemblage.
[Fig. 15]La Figure 15 est une vue en perspective montrant le positionnement du câble d'actionnement dans la vertèbre tubulaire proximale de la structure de flexion.
[Fig. 16]La Figure 16 est une vue côté intérieur en perspective montrant le positionnement du câble d'actionnement dans la vertèbre tubulaire proximale de la structure de flexion.
[Fig. 17]La Figure 17 est une vue en perspective montrant le tube d'insertion et la structure de flexion en position assemblée.

### Description des modes de réalisation

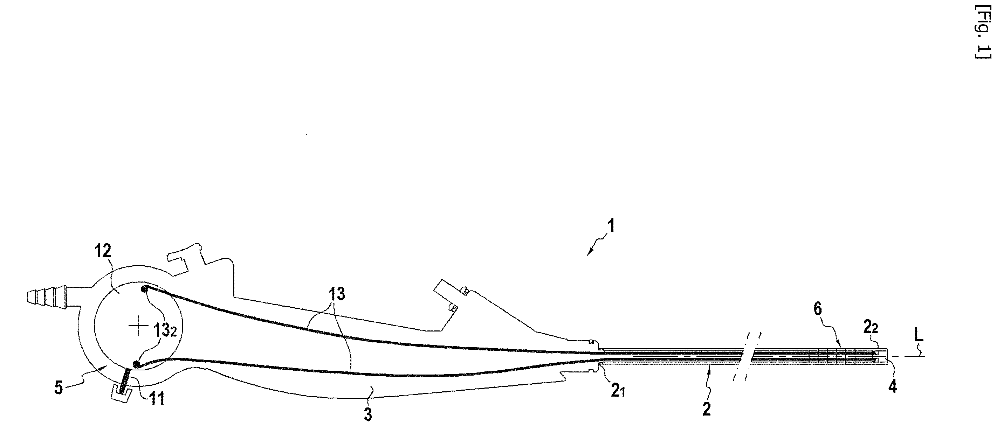

La Figure 1 illustre à titre d'exemple, un dispositif médical 1 du type endoscope ou cathéter 1 au sens général conçu pour accéder à l'intérieur d'un corps comme une cavité ou un canal par exemple. De manière classique, un dispositif médical 1 du type endoscope ou cathéter comporte un tube d'insertion 2 présentant d'un côté, une partie proximale 2₁ reliée à une poignée de commande 3 et du côté opposé, une partie distale 2₂, qui est équipée d'une tête distale 4. Le tube d'insertion 2 est fixé de manière temporaire ou définitive sur la poignée de commande 3. Ce tube d'insertion 2 qui présente une longueur et une flexibilité plus ou moins importante est destiné à être introduit dans une voie d'accès naturelle ou artificielle en vue d'effectuer diverses opérations ou fonctions à des fins thérapeutiques, chirurgicales ou de diagnostic. Le tube d'insertion 2 est réalisé en un matériau semi-rigide et présente une longueur adaptée à la longueur du conduit à inspecter et pouvant être comprise entre 5 cm et 2 m. Le tube d'insertion 2 présente diverses formes de section transversale telle que carrée, ovale ou circulaire. Ce tube d'insertion 2 qui est en contact avec les tissus, les organes humains ou des appareillages médicaux (trocarts ou sondes), relève essentiellement d'un usage unique ou multiple d'un patient voire d'un usage réutilisable après décontamination, désinfection ou stérilisation.

Selon un exemple préféré de réalisation, le dispositif médical 1 conforme à l'invention est un endoscope comportant un système de vision apte à éclairer et à ramener une image de la partie distale du tube d'insertion 2. L'endoscope comporte ainsi un système de vision monté à l'intérieur de la poignée de commande 3 et pénétrant à l'intérieur du tube d'insertion 2 jusqu'à la tête distale 4. De même, le dispositif médical 1 comporte également à l'intérieur du tube d'insertion 2, un canal opérateur ou de travail s'étendant de la poignée de commande 3 à la tête distale 4 pour permettre l'amenée de divers outillages et/ou de fluides et/ou l'aspiration de fluides.

De manière classique, le dispositif médical 1 comporte également un mécanisme de commande 5 permettant d'orienter la tête distale 4 par rapport à l'axe longitudinal L du tube d'insertion 2. A cet effet, le tube d'insertion 2 comporte en amont de la tête distale 4, une structure de flexion, de pliage ou de béquillage 6 conforme à l'invention permettant l'orientation de la tête distale 4 par rapport à l'axe longitudinal L du tube d'insertion 2.

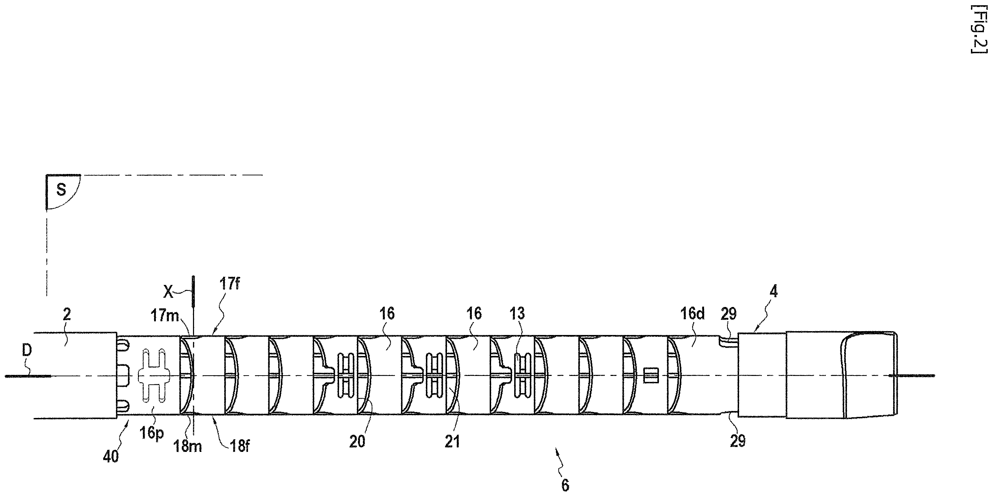

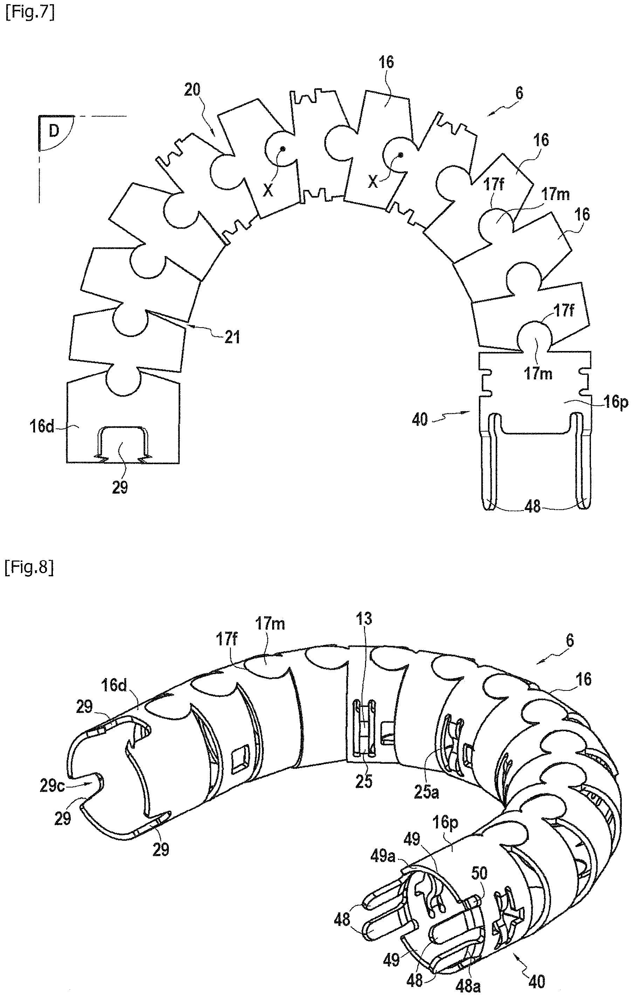

Le mécanisme de commande 5 peut être réalisé de toute manière appropriée de manière que la tête distale 4 puisse être déplacée entre une position de repos dans laquelle le tube d'insertion 2 est rectiligne (Figure 2) et une position béquillée dans laquelle la partie de béquillage 6 est courbée (Figures 7, 8). A titre d'exemple non limitatif, le mécanisme de commande 5 peut correspondre au mécanisme de commande décrit dans le brevet FR 3 047 887. A cet effet, le mécanisme de commande 5 comporte un levier de commande manuelle 11 entraînant en rotation au moins une pièce pivotante comme une poulie 12 sur laquelle est fixé au moins un câble d'actionnement 13 monté à l'intérieur du tube d'insertion 2 pour être fixée au niveau de la tête distale 4. Typiquement, chaque câble d'actionnement 13 est entouré par une gaine 13g sur au moins une partie de sa longueur.

Selon un exemple préféré de réalisation illustré par les Figures, la structure de flexion 6 comporte un tube 15 découpé par des traits de découpe T provenant d'un faisceau d'énergie pour réaliser des bagues ou des vertèbres tubulaires 16 imbriquées les unes dans les autres par une première série de zones découpées 17m, 17f formant des pivots de rotation et par une deuxième série de zones découpées 18m, 18f formant également des pivots de rotation. Les zones découpées 17m, 17f de la première série s'étendent diamétralement opposées par rapport aux zones découpées 18m, 18f de la deuxième série pour former un premier axe de pivotement X. Ainsi, deux vertèbres tubulaires 16 voisines sont adaptées pour pivoter relativement entre elles autour d'un premier axe de pivotement X passant par un pivot de rotation 17m, 17f de la première série et par un pivot de rotation 18m, 18f de la deuxième série. Par convention, il est considéré que le tube 15 possède, au repos, un plan de symétrie S dans lequel sont situés les premiers axes de pivotement X s'étendant parallèlement entre eux.

Avantageusement, chaque série de zones découpées comporte alternativement des zones découpées mâles 17m, 18m emboitées dans des zones découpées femelles respectivement 17f, 18f. Ainsi, une vertèbre tubulaire 16 comporte deux zones découpées mâles 17m, 18m situées diamétralement opposées coopérant avec deux zones découpées femelles 17f, 18f aménagées de manière également diamétralement opposées dans la vertèbre tubulaire 16 adjacente. Bien entendu, les formes découpées mâles 17m, 18m sont congruentes ou complémentaires aux formes découpées femelles 17f, 18f pour permettre une rotation relative entre deux vertèbres tubulaires 16 voisines et un emboitement entre deux vertèbres tubulaires 16 voisines.

Il doit être compris que les zones découpées 17m, 17f, 18m, 18f formant des pivots de rotation coopèrent entre elles par leur épaisseur. En d'autres termes, une zone découpée mâle 17m, 18m est en contact ou en appui par son bord avec le bord de la zone découpée femelle 17f, 18f. Tel que cela ressort de l'exemple de réalisation illustré sur les Figures 2 à 10, les formes découpées mâles 17m, 18m sont des portions de disques d'étendue angulaire supérieure à 180° (par exemple comprise entre 200° et 300°) pour être emboitées dans des portions de bagues ou de coussinets.

Il doit être compris que la structure de flexion 6 est obtenue à partir d'un unique tube 15 s'étendant selon un axe longitudinal rectiligne L et possédant une section droite de préférence circulaire. Le tube 15 possède une épaisseur adaptée pour pouvoir être découpé sur toute son épaisseur par un faisceau d'énergie de tous types connus en soi, tel un faisceau plasma, un jet d'eau ou de préférence par un faisceau laser. Par exemple, le tube 15 est découpé par laser CO2 ou YAG. Le tube 15 présente une épaisseur de matériau comprise entre 0,05 et 2 mm. Le tube 15 possède un diamètre compris par exemple entre 1 mm et 20 mmm.

De même, le tube 15 est réalisé en un matériau adapté pour être découpé par un faisceau d'énergie tout en présentant les caractéristiques mécaniques de flexion et de résistance mécanique nécessaires à la structure de flexion 6. Par exemple, le tube 15 est réalisé en acier inoxydable.

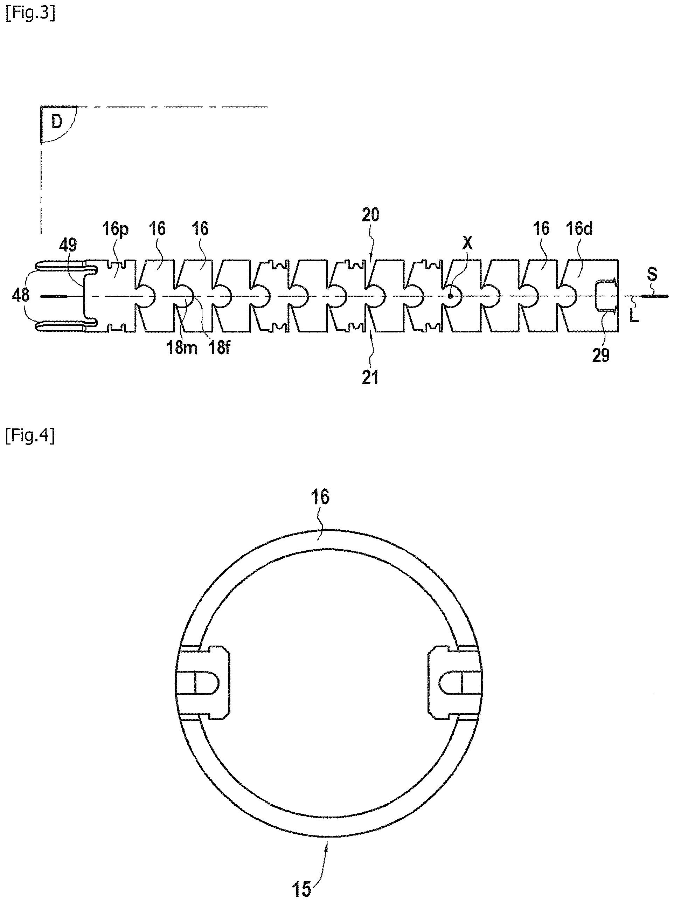

Tel que cela ressort des Figures 2 et 3, les traits de découpe T sont aménagés pour former dans le tube, une série de vertèbres tubulaires 16 juxtaposées les unes aux autres, avec une vertèbre tubulaire dite distale 16d et une vertèbre tubulaire dite proximale 16p. La structure de flexion 6 comporte ainsi à son extrémité distale, une vertèbre tubulaire distale 16d destinée à être fixée à la tête de distale 4 du dispositif médical et à son extrémité proximale, une vertèbre tubulaire proximale 16p destinée à être fixée à l'extrémité distale du tube d'insertion 2. Par exemple, la structure de flexion 6 montée entre le tube 2 et la tête distale 4 est insérée à l'intérieur d'une gaine ou d'une enveloppe de protection. De préférence, en dehors de la vertèbre tubulaire distale 16d et de la vertèbre tubulaire proximale 16p, les vertèbres tubulaires 16 présentent toutes la même largeur prise selon l'axe longitudinal L. Par exemple, ces vertèbres tubulaires 16 présentent une largeur comprise par exemple entre 1 et 25 mm.

Tel que cela ressort de l'exemple de réalisation illustré sur les Figures 2 à 10, des traits de découpe T sont aménagés dans le tube 15 pour réaliser une première série d'entailles 20 et une deuxième série d'entailles 21 découpées dans le tube en s'étendant symétriquement de part et d'autre du plan de symétrie S pour former des zones de flexion pour les vertèbres tubulaires 16. En d'autres termes, les entailles 20, 21 sont aménagées pour autoriser le pivotement des vertèbres tubulaires entre elles autour du premier axe de pivotement X, selon un plan diamétral D de flexion perpendiculaire au premier axe de pivotement X. La structure tubulaire 6 comporte ainsi d'un côté du premier plan de symétrie S, les entailles 20 de la première série séparant deux à deux les vertèbres tubulaires 16 et de l'autre côté du plan de symétrie S, les entailles 21 de la deuxième série séparant deux à deux les vertèbres tubulaires 16.

De préférence, les entailles 20 de la première série sont réalisées selon une plage d'extension angulaire identique. De même, les entailles 21 de la deuxième série sont réalisées selon une plage d'extension angulaire identique. Avantageusement, les entailles 20 de la première série et les entailles 21 de la deuxième série sont réalisées selon une plage d'extension angulaire identique. Avantageusement, les entailles 20 de la première série et les entailles 21 de la deuxième série sont centrées par rapport au plan diamétral D passant par le diamètre du tube et perpendiculaire au plan de symétrie S.

Il ressort de la description qui précède que les entailles 20, 21 sont aménagées dans le tube 15 avec un enlèvement de matière. De préférence, les entailles 20 de la première série sont réalisées avec une découpe identique en dimensions et en forme. De même, les entailles 21 de la deuxième série sont réalisées avec une découpe identique en dimensions et en forme. Avantageusement, les entailles 20 de la première série et les entailles 21 de la deuxième série sont réalisées avec une découpe identique en dimensions et en forme. Les entailles 20 de la première série et les entailles 21 de la deuxième série sont réalisées par des découpes de forme oblongue ou effilée, adaptées pour permettre un pivotement relatif entre deux vertèbres tubulaires 16 adjacentes selon les premiers axes de pivotement X.

Tel que cela ressort plus précisément de la Figure 3, chaque entaille 20, 21 entre deux vertèbres tubulaires 16 adjacentes résulte d'une découpe aménagée uniquement dans le bord externe d'une vertèbre tubulaire 16. Bien entendu, chaque entaille 20, 21 entre deux vertèbres tubulaires 16 adjacentes peut résulter d'une découpe aménagée dans le bord externe de deux vertèbres tubulaires 16 voisines.

Dans les exemples de réalisation illustré aux Figures 2 à 10, la structure tubulaire 6 comporte une première série d'entailles 20 et une deuxième série d'entailles 21 pour obtenir la flexion de la structure de flexion dans les deux sens de la direction perpendiculaire au premier axe de pivotement X. Bien entendu, il peut être prévu de réaliser une structure tubulaire comportant une unique série d'entailles dans le cas où la structure tubulaire 6 est pourvue d'un unique câble d'actionnement 13 pour obtenir la flexion de la structure de flexion dans un seul sens de la direction perpendiculaire au premier axe de pivotement X. Par ailleurs, dans l'exemple de réalisation illustré aux Figures 2 à 8, les vertèbres tubulaires 16 sont articulées selon uniquement le premier axe de pivotement X afin d'obtenir la flexion de la structure de flexion dans un plan perpendiculaire à savoir le plan diamétral de flexion D.

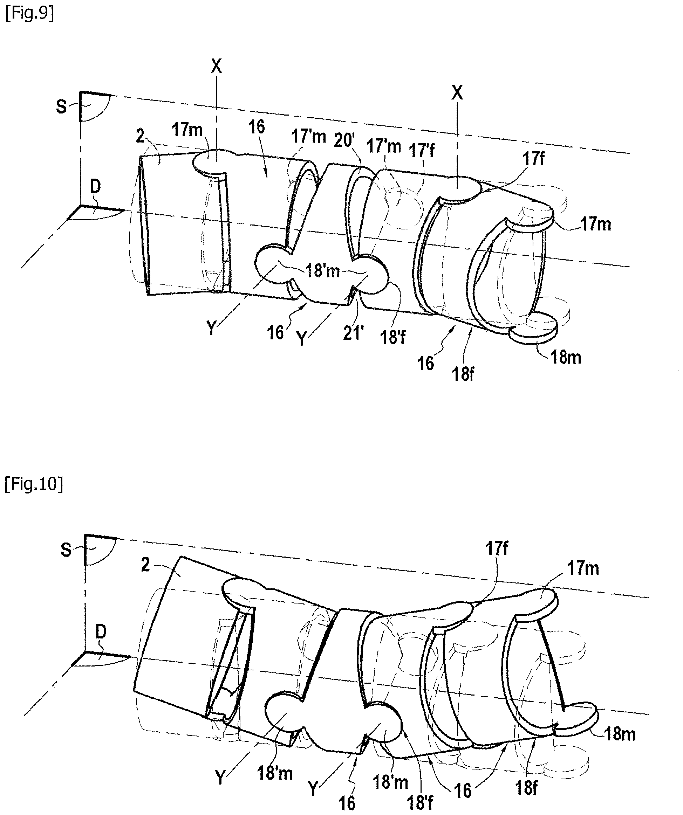

Selon une autre variante de réalisation, il peut être prévu de réaliser une structure tubulaire 6 adaptée pour permettre une flexion dans deux plans perpendiculaires entre eux à l'aide de trois ou de quatre câbles d'actionnement 13 en vue d'obtenir le déplacement gauche-droit et haut-bas de la tête distale 4. Les Figures 9 et 10 illustrent un tel exemple de réalisation pour lequel le tube 15 est découpé pour comporter une troisième série de zones découpées 17'm, 17'f formant des pivots de rotation et par une quatrième série de zones découpées 18'm, 18'f formant des pivots de rotation s'étendant diamétralement opposée par rapport à la troisième série et selon un plan perpendiculaire au premier plan de symétrie S à savoir le plan diamétral de flexion D. Ainsi, deux vertèbres tubulaires 16 voisines équipées des zones découpées 17'm, 17'f de la troisième série et des zones découpées 18'm, 18'f de la quatrième série sont adaptées pour pivoter relativement entre elles autour d'un deuxième axe de pivotement Y passant par un pivot de rotation 17'm, 17'f de la troisième série et par un pivot de rotation 18'm, 18'f de la quatrième série, ce deuxième axe de pivotement Y étant perpendiculaire par rapport au premier axe de pivotement X.

Les zones découpées 17'm, 17'f de la troisième série et les zones découpées 18'm, 18'f de la quatrième série peuvent être réalisées sur des vertèbres tubulaires 16 déjà pourvues des zones découpées 17m, 17f de la première série et des zones découpées 18m, 18f de la deuxième série ou sur des vertèbres tubulaires 16 dépourvues des zones découpées 17m, 17f de la première série et des zones découpées 18m, 18f de la deuxième série, comme illustré à titre d'exemple sur les Figures 9 et 10. Dans ce dernier cas, l'alternance des vertèbres tubulaires 16 entre celles permettant une rotation selon le premier axe de pivotement X et celles permettant une rotation selon le deuxième axe de pivotement Y est choisie pour obtenir le rayon de flexion souhaité.

Des traits de découpe sont aménagées pour délimiter entre les vertèbres tubulaires 16, une troisième série d'entailles 20' et une quatrième série d'entailles 21' découpées dans le tube en s'étendant symétriquement de part et d'autre du plan diamétral de flexion D pour former des zones de flexion pour les vertèbres tubulaires afin d'obtenir la flexion de la structure de flexion dans un plan de flexion perpendiculaire au plan diamétral de flexion D c'est-à-dire dans le plan de symétrie S (Figure 10).

Cette troisième série de zones découpées 17'm, 17'f et la quatrième série de zones découpées 18'm, 18'f présentent les mêmes caractéristiques que les zones découpées 17m, 17f de la première série et des zones découpées 18m, 18f de la deuxième série. Dans l'exemple illustré, les zones découpées 17'm, 17'f de la troisième série et les zones découpées 18'm, 18'f de la quatrième série présentent la même forme de réalisation mais il clair que les zones découpées 17'm, 17'f de la troisième série et les zones découpées 18'm, 18'f de la quatrième série peuvent être réalisées différemment des zones découpées 17m, 17f de la première série et des zones découpées 18m, 18f de la deuxième série.

Les zones découpées des troisième et quatrième séries 17'm, 17'f, 18'm, 18'f formant des pivots de rotation coopèrent entre elles par leur épaisseur. De même, les zones découpées des troisième et quatrième séries 17'm, 17'f, 18'm, 18'f formant des pivots de rotation comporte alternativement des zones découpées mâles 17'm, 18'm emboitées dans des zones découpées femelles 17'f, 18'f.

D'une manière générale, il ressort de la description qui précède que le tube 15 est découpé par des traits de découpe T d'un faisceau d'énergie pour réaliser des vertèbres tubulaires 16 imbriquées les unes dans les autres par des zones découpées 17m, 17f, 18m, 18f, 17'm, 17'f, 18'm, 18'f formant des pivots de rotation selon deux axes de pivotement X, Y, perpendiculaires entre eux. Des traits de découpe sont aménagées pour délimiter entre les vertèbres tubulaires 16 des entailles 20, 21, 20', 21' découpées dans le tube pour former des zones de flexion pour les vertèbres tubulaires afin d'obtenir la flexion de la structure de flexion dans au moins un plan de flexion D, S perpendiculaire aux axes de pivotement X, Y.

La structure tubulaire 6 comporte ainsi une série de vertèbres tubulaires 16 emboitées les unes dans les autres, directement en sortie de découpe, sans nécessité d'opérations de montage des vertèbres tubulaires 16 entre elles. Les vertèbres tubulaires 16 sont ainsi assemblées ensemble par emboitement les unes dans les autres. Les entailles 20, 21, 20', 21' forment des zones de flexion afin d'obtenir la flexion de la structure de flexion dans au moins un plan diamétral D, S de flexion perpendiculaire à l'axe de pivotement X, Y (Fig. 7 à 10). Cette structure tubulaire 6 présente un coût réduit et peut présenter des diamètres réduits dans la mesure où elle ne nécessite aucune opération de montage des vertèbres tubulaires 16 entre elles.

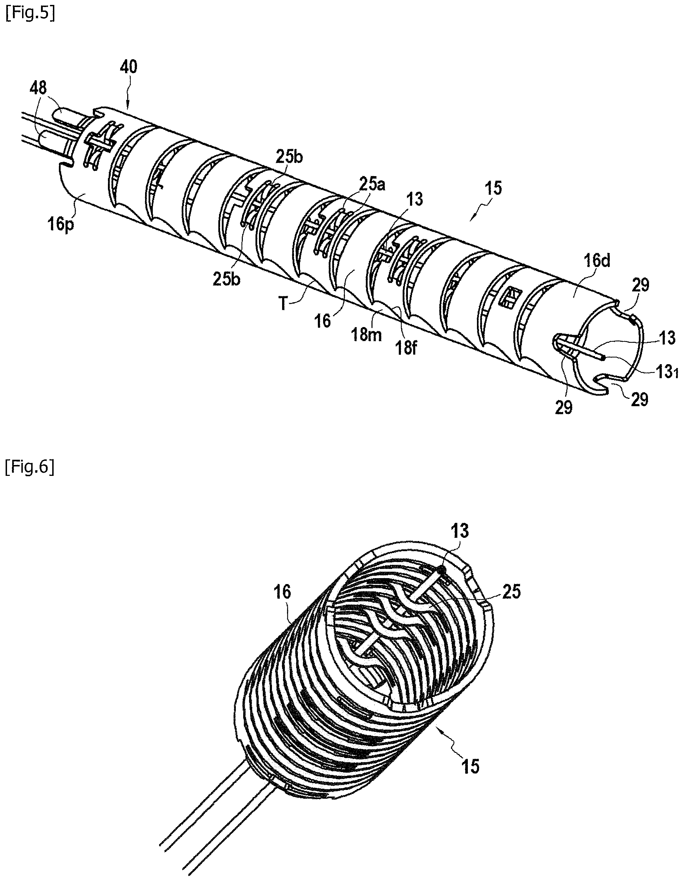

Selon une caractéristique avantageuse de réalisation, certaines des vertèbres tubulaires 16 comportent au moins une languette 25 découpée du tube 15 par deux lignes de découpe parallèles 25a, en restant attachée au tube à ses extrémités par des zones d'attache 25b. Chaque languette 25 est repoussée à l'intérieur du tube pour constituer un œil de guidage pour le câble d'actionnement 13. Par exemple, chaque languette 25 possède ainsi, à partir des zones d'attache 25b, un segment incurvé vers l'intérieur du tube et se raccordant ensemble par une partie centrale concave (Figure 6).

Selon une caractéristique avantageuse de réalisation, chaque languette 25 est réalisée selon une plage d'extension angulaire centrée sur la plage d'extension des entailles voisines 20, 21, 20', 21'. Ainsi, les languettes 25 sont centrées par rapport au plan de flexion. Pour chaque câble d'actionnement 13, plusieurs languettes 25 sont aménagées selon une génératrice du tube pour assurer le guidage en translation du câble d'actionnement. Dans l'exemple illustré aux Figures 2 à 8, chaque câble d'actionnement 13 est guidé par trois languettes 25. Ainsi, trois vertèbres tubulaires 16 comportent deux languettes 25 disposées symétriquement de part et d'autre du plan de symétrie S en étant centrées par rapport au plan diamétral D. Bien entendu, les languettes 25 peuvent être aménagées sur un nombre différent de vertèbres tubulaires 16. De même dans l'exemple illustré, les vertèbres tubulaires 16 pourvues des languettes 25 de guidage sont séparés deux à deux par une vertèbre tubulaire 16 non munie d'une languette de guidage. Il est clair que les languettes 25 peuvent être réalisées sur les vertèbres tubulaires 16 selon une distribution différente. Il doit être compris qu'une série de languettes 25 est réalisée le long d'une génératrice du tube pour chacun des câbles d'actionnement 13 équipant la structure de flexion 6.

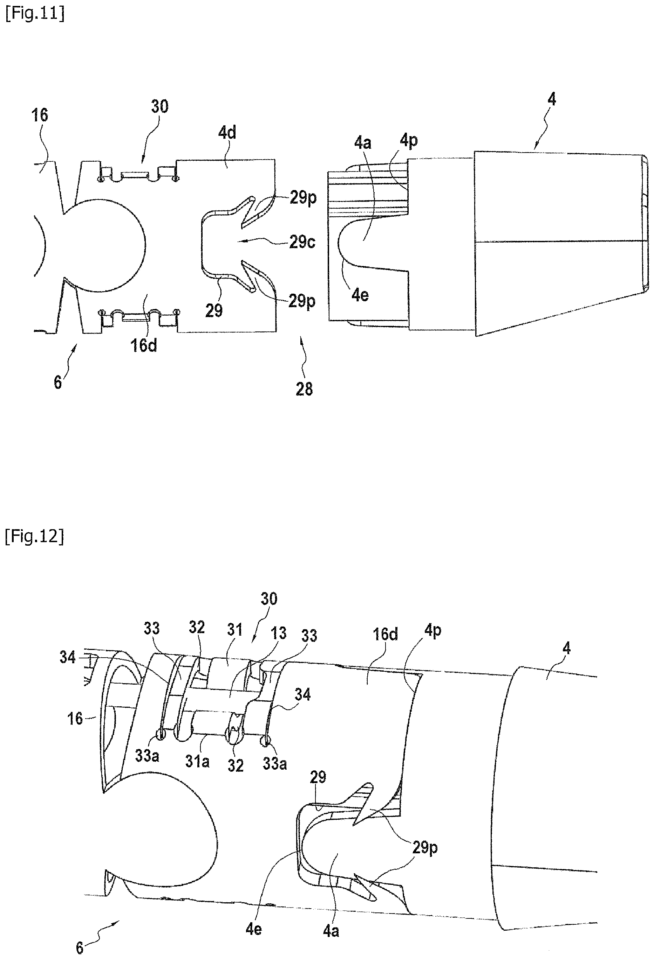

Selon une autre caractéristique avantageuse de réalisation, la structure de flexion 6 comporte une vertèbre tubulaire distale 4d pourvue d'un système d'assemblage 28 avec la tête distale 4 comportant au moins deux découpes 29 d'insertion pour des doigts 4a de la tête distale 4. Ces découpes 29 sont aménagées dans la vertèbre tubulaire distale 16d en s'ouvrant chacune à la partie distale de la vertèbre tubulaire distale 16d par un canal d'engagement 29c. Dans l'exemple illustré à la Figure 8, trois découpes 29 sont aménagées délimitant trois canaux d'engagement 29c répartis régulièrement selon la circonférence de la vertèbre tubulaire distale 16d. Chaque canal 29c possède à partir de la partie distale de la vertèbre tubulaire distale 16d, une section tronconique qui se rétrécie en direction du fond du canal 29c. La tête distale 4 comporte également au moins deux doigts 4a et dans l'exemple illustré, trois doigts 4a s'étendant en saillie à partir d'un bord proximal 4p de la tête distale 4, selon une répartition angulaire identique à la répartition des découpes 29. Chaque doigt 4a est aménagé pour présenter en direction d'une extrémité arrondie 4e, une section tronconique complémentaire à la section tronconique du canal d'engagement 29c et adaptée pour s'engager dans le canal 29c jusqu'à ce que le bord proximal 4p de la tête distale vienne en butée contre l'extrémité distale de la vertèbre tubulaire distale 16d (Figure 12). L'engagement des doigts 4a de la tête distale 4 dans les découpes 29 de la vertèbre tubulaire distale 4d assure une orientation radiale entre la structure de flexion 6 et la tête distale 4 et un blocage en rotation entre ces deux pièces.

Selon une autre caractéristique du système d'assemblage 28, au moins une découpe 29 et dans l'exemple illustré, une découpe 29 est aménagée pour présenter deux pointes 29p de blocage pour un doigt 4a de la tête distale, s'étendant de part et d'autre du canal d'engagement 29c et orientées sensiblement vers le fond de la découpe 29. Lors de l'engagement du doigt 4a à l'intérieur du canal d'engagement 29c lors d'un mouvement de translation, les pointes 29p pénètrent dans les bords du doigt 4a assurant le blocage de la tête distale 4 par rapport à la structure de flexion 6, dans le sens d'extraction. La coopération entre les pointes 29p et les doigts 4a assure l'anti-extraction axiale de la tête distale par rapport à la structure de flexion 6. La tête distale 4 peut ainsi être fixée facilement et de manière sûre, à la structure de flexion 6 conforme à l'invention. De plus, comme expliqué précédemment, cette vertèbre tubulaire distale 16d pourvue du système d'assemblage 28 peut être obtenue directement en sortie de découpe, sans la mise en œuvre d'étapes supplémentaires de fabrication.

Selon une autre caractéristique avantageuse de réalisation, la structure de flexion 6 comporte une vertèbre tubulaire distale 16d sur laquelle est soudée une extrémité 13₁ d'au moins un et dans l'exemple illustré, de deux câbles d'actionnement 13 comme illustré à la Figure 5. Bien entendu dans le cas où la structure de flexion 6 est pourvue de trois ou de quatre câbles d'actionnement 13, les extrémités des câbles d'actionnement peuvent être soudées sur la vertèbre tubulaire distale 16d. Classiquement, le câble d'actionnement 13 est réalisé en acier inoxydable. Le soudage de l'extrémité du câble d'actionnement 13 peut être réalisé de toute manière appropriée comme par la technique de soudure par point (principe utilisant le principe de la soudure par résistance) ou par soudage laser. Bien entendu, les extrémités 13₂ des câbles d'actionnement 13 sont fixées à la poulie 12 à l'aide de tous types de fixation adaptés.

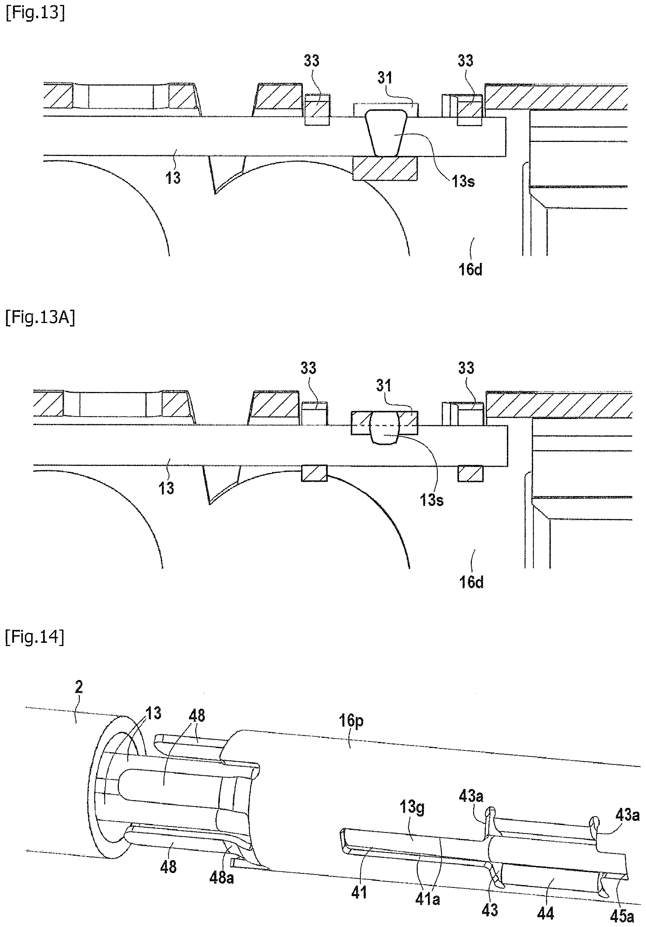

Selon une caractéristique avantageuse de réalisation illustrée plus précisément aux Figures 12 et 13, la structure de flexion 6 comporte une vertèbre tubulaire distale 16d pourvu d'un système de positionnement 30 du câble d'actionnement 13 pour permettre son soudage correct sur la vertèbre tubulaire distale 16d. Ce système de positionnement 30 comporte une languette d'appui 31 radiale découpée du tube par deux lignes de découpe parallèles 32, en restant attachée au tube à ses deux extrémités par des zones d'attache 31a. La languette d'appui 31 est donc repoussée à l'intérieur de la vertèbre tubulaire distale 16d pour permettre le positionnement du câble d'actionnement 13 sur la surface externe de la languette d'appui 31 de sorte que le câble d'actionnement 13 est accessible à partir de l'extérieur du tube 15. De manière plus précise, le câble d'actionnement 13 est sollicité en appui sur cette languette 31 par deux contre languettes 33 s'étendant radialement de part et d'autre de la languette d'appui 31 en étant chacune découpée du tube par deux lignes de découpe 34 parallèles, et en restant attachée au tube à ses deux extrémités par des zones d'attache 33a. Ces deux contre languettes 33 assurent un bon contact du câble d'actionnement 13 avec la languette d'appui 31 permettant d'assurer un soudage efficace du câble d'actionnement 13 sur la vertèbre tubulaire distale 16d. A titre d'exemple, la figure 13 montre la réalisation d'une soudure 13s avantageusement par transparence permettant de fusionner le câble d'actionnement 13 à la languette d'appui 31 du tube 15. De plus, comme expliqué précédemment, cette vertèbre tubulaire distale 16d pourvue du système de positionnement 30 peut être obtenue directement en sortie de découpe, sans la mise en œuvre d'étapes supplémentaires de fabrication.

La Figure 13A illustre une variante alternative de positionnement du câble d'actionnement 13 pour permettre son soudage correct sur la vertèbre tubulaire distale 16d. Selon cette variante, le câble d'actionnement 13 est supporté par les deux contre languettes 33 qui s'étendent radialement de part et d'autre de la languette d'appui 31. La languette d'appui 31 est repoussée à l'intérieur de la vertèbre tubulaire distale pour venir par sa surface interne, en appui sur le câble d'actionnement 13. La languette d'appui 31 est ainsi déformée pour positionner correctement le câble d'actionnement 13 avant soudure. Une soudure 13s par transparence permet ainsi de fusionner le câble d'actionnement 13 à la languette d'appui 31 du tube 15. Ainsi, le câble d'actionnement 13 est positionné avant soudure entre les contre languettes 33 et la languette d'appui 31, et au moins une soudure 13s permet de fusionner le câble d'actionnement 13 à la languette d'appui 31.

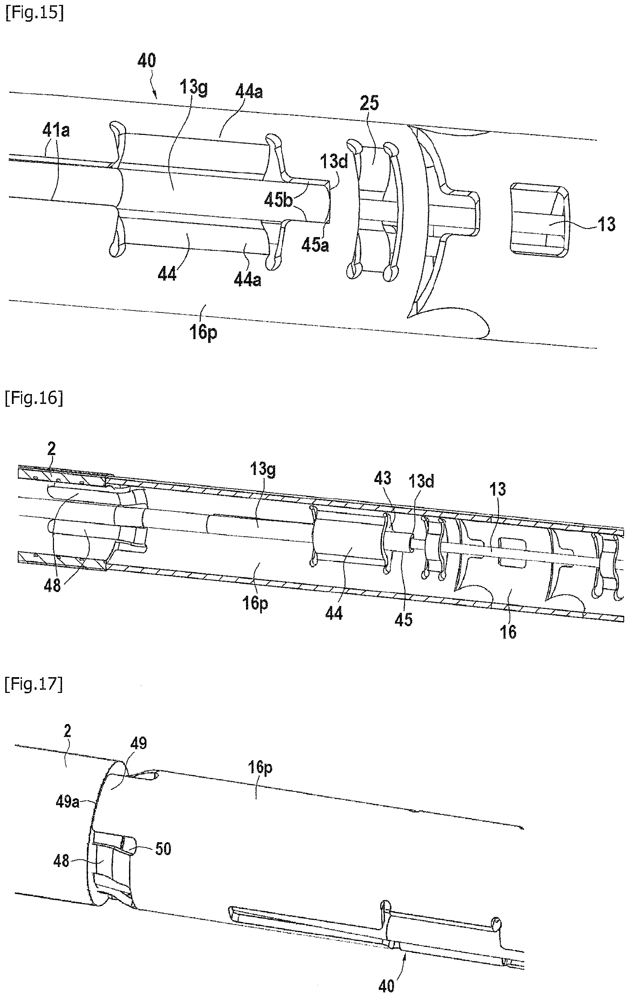

Selon l'invention illustrée plus particulièrement aux Figures 14 à 16, la vertèbre tubulaire proximale 16p comporte pour chaque câble d'actionnement 13, un système de blocage axial et radial 40 pour la gaine 13g entourant chaque câble d'actionnement 13. Ce système de blocage axial et radial 40 comporte une découpe 41 aménagée axialement dans la vertèbre tubulaire proximale 16p, parallèlement à son axe longitudinal. Cette découpe 41 est de forme allongée et se trouve délimitée par deux bords dits d'arrêt 41a s'étendant parallèlement entre eux et à l'axe longitudinal de la vertèbre tubulaire proximale 16p. La largeur de cette découpe entre ses bords d'arrêt 41a est déterminée pour permettre un engagement partiel de la gaine 13g à l'intérieur de la découpe 41. En d'autres termes, cette découpe 41 est aménagée pour délimiter deux bords d'arrêt 41a limitant l'engagement radial de la gaine 13g à l'intérieur de la découpe. Tel que cela ressort plus précisément des Figures 14 et 15, la gaine 13g est en contact avec les bords d'arrêt 41a selon deux de ses génératrices en considérant que la partie de la gaine située entre ces deux génératrices est insérée à l'intérieur de la découpe 41. Par exemple, lorsque la gaine 13g est en butée contre les bords d'arrêt 41a, la gaine 13g par sa génératrice la plus extérieure, est sensiblement tangente avec la surface extérieure de la vertèbre tubulaire proximale 16p. Il est à noter qu'une telle disposition libère de la place à l'intérieur de la structure de flexion 6.

Cette découpe 41 débouche à sa partie distale, dans une lumière 43 délimitée par au moins deux lignes de découpe 43a aménagées radialement dans la vertèbre tubulaire proximale 16p pour délimiter entre elles, une languette d'appui 44 attachée à la vertèbre tubulaire proximale 16p, par ses extrémités à l'aide de zones d'attache 44a. Cette languette d'appui 44 est repoussée à l'intérieur de la vertèbre tubulaire proximale 16p pour permettre le positionnement de la gaine 13g du câble d'actionnement sur la surface externe de la languette d'appui 44, entre la languette d'appui 44 et le corps de la vertèbre tubulaire proximale 16p. Ainsi, la languette d'appui 44 est aménagée pour solliciter radialement la gaine 13g en appui contre les deux bords d'arrêt 41a de la découpe 41.

Selon l'invention, cette lumière 43 est bordée par un rebord d'arrêt axial 45a pour la gaine 13g du câble d'actionnement 13. La gaine 13g présente une extrémité distale 13d destinée à être en appui sur le rebord d'arrêt axial 45a. Il doit être compris qu'au-delà de cette extrémité distale 13d de la gaine 13g, le câble d'actionnement 13 est dépourvu de la gaine 13g. Avantageusement, le rebord d'arrêt axial 45a est réalisé par le bord radial d'une entaille 45 aménagée axialement dans la vertèbre tubulaire proximale 16p, en débouchant du côté proximal, dans la lumière 43. Cette entaille 45 se trouve délimitée par deux bords d'appui 45b s'étendant parallèlement entre eux et à l'axe longitudinal de la vertèbre tubulaire proximale 16p. Ces deux bords d'appui 45b sont raccordés entre eux du côté distal, par le rebord d'arrêt axial 45a. Cette entaille 45 est aménagée pour permettre un engagement partiel de la gaine 13g à l'intérieur de l'entaille 45 et le positionnement de l'extrémité distale 13d de la gaine 13g contre le rebord d'arrêt axial 45a. La gaine 13g est ainsi en butée par sensiblement la moitié de son extrémité distale 13d contre le rebord d'arrêt axial 45a.

Selon une caractéristique avantageuse de mise en œuvre, il est à noter que lorsque la gaine 13g est en butée contre le rebord d'arrêt axial 45a, il est possible de souder la gaine 13g à la vertèbre tubulaire proximale 16p de manière à bloquer en translation la gaine 13g. A cet effet, une soudure est réalisée par exemple entre l'extrémité distale 13d de la gaine 13g d'une part et le rebord d'arrêt axial 45a et/ou les bords d'appui 45b et/ou les bords d'arrêt 41a de la vertèbre tubulaire proximale 16p d'autre part.

Il est à noter que la languette d'appui 44 sollicite également radialement la gaine 13g pour venir en contact contre les deux bords d'appui 45b, assurant le positionnement de l'extrémité distale 13d de la gaine 13g contre le rebord d'arrêt axial 45a. Typiquement, l'entaille 45 et la découpe 41 sont aménagées dans le prolongement l'une de l'autre. Par exemple, lorsque la gaine 13g est en butée contre les bords d'appui 45b, la gaine 13g par sa génératrice la plus extérieure, est sensiblement tangente avec la surface extérieure de la vertèbre tubulaire proximale 16p.

Il est à noter que le rebord d'arrêt axial 45a peut ne pas être délimité par l'entaille 45 tel qu'illustré sur les dessins mais par le bord découpé distal délimitant la lumière 43. De préférence, la vertèbre tubulaire proximale 16p est pourvue entre le système de blocage axial et radial 40 et son extrémité distale, d'une languette 25 aménagée pour assurer le guidage en translation du câble d'actionnement 13.

Il ressort de la description qui précède qu'un tel système de blocage axial et radial 40 permet d'assurer un positionnement correct du câble d'actionnement 13 limitant les frottements à l'intérieur de la gaine 13g. Le système de blocage 40 permet de positionner, sans courbure, le câble d'actionnement 13 afin qu'il se trouve aligné avec sa partie qui est guidée en translation par les languettes 25. Ce système de blocage 40 participe également à la tenue mécanique radiale entre le tube d'insertion 2 et la structure de flexion 6. Par ailleurs, le blocage axial de la gaine 13g contre le rebord d'arrêt axial 45a constitue une butée robuste permettant de passer des efforts importants dans le câble d'actionnement.

Selon une caractéristique avantageuse de réalisation, la vertèbre tubulaire proximale 16p comporte à sa partie proximale, des languettes 48 de liaison s'étendant axialement en présentant une flexibilité radiale pour s'engager dans la partie distale du tube d'insertion 2 et assurer l'assemblage entre le tube d'insertion 2 et la structure de flexion 6. Les languettes 48 sont distribuées sur la circonférence de la vertèbre tubulaire proximale 16p de manière régulière ou non. Par exemple, la vertèbre tubulaire proximale 16p comporte deux paires de deux languettes 48 aménagées de manière diamétralement opposée. Il est à noter que les deux languettes 48 d'une paire sont écartées de manière à permettre le positionnement entre elles et le guidage d'un câble d'actionnement 13 comme illustré à la Figure 16.

Chaque languette 48 présente une déformation radiale 48a réalisée par exemple par des plis, de manière que la languette s'étend sensiblement parallèlement à l'axe longitudinal de la vertèbre tubulaire proximale 16p mais en étant décalée vers l'intérieur de la vertèbre tubulaire proximale. Le décalage radial des languettes 48 permet l'engagement des languettes dans la partie distale du tube d'insertion 2 présentant un diamètre sensiblement identique au diamètre de la vertèbre tubulaire proximale 16p. Tel que cela ressort plus précisément de la Figure 17, la vertèbre tubulaire proximale 16p et le tube d'insertion 2 sont ainsi affleurant en position assemblée. Il est à noter que l'assemblage par des languettes offre l'avantage de présenter un encombrement limité tout en évitant l'ajout d'une pièce supplémentaire de liaison venant rajouter une épaisseur.

De préférence, les languettes 48 sont aménagées de manière à laisser subsister des dents 49 entre deux languettes voisines. Les dents 49 sont détachées des dents 49 par des découpures 50 contribuant à la flexibilité des languettes 48. Dans l'exemple illustré, la vertèbre tubulaire proximale 16p comporte deux dents 49 s'étendant de façon diamétralement opposée et de part d'autre desquelles sont situées les paires de languettes 48. Les dents 49 s'étendent axialement à partir de la vertèbre tubulaire proximale 16p en retrait de l'extrémité des languettes 48, et en présentant des bords d'extrémité 49a délimitant ensemble, le bord proximal de la vertèbre tubulaire proximale 16p. Les languettes 48 peuvent ainsi être engagées à l'intérieur du tube d'insertion 2 jusqu'à ce que les bords d'extrémité 49a des dents viennent en butée sur le bord de l'extrémité distale du tube d'insertion 2 (Figure 17).

Il ressort de la description qui précède que l'objet de l'invention concerne un tube d'insertion 2 d'un dispositif médical 1 comportant une structure de flexion 6 fixée à son extrémité proximale au tube d'insertion 2 et à son extrémité distale, à la tête distale 4. Avantageusement, la tête distale 4 est assemblée à la structure de flexion 6 par le système d'assemblage 28 du type à ancrage par pointes tandis que le tube d'insertion 2 est assemblé à la structure de flexion à l'aide des languettes de liaison 48. Comme expliqué précédemment, les vertèbres tubulaires proximale 16p et distale 16d telles que décrite ci-dessus peuvent être obtenues directement en sortie de découpe, sans la mise en œuvre d'étapes supplémentaires de fabrication.

L'invention n'est pas limitée aux exemples décrits et représentés, l'invention étant limitée seulement par l'objet des revendications annexées.

## Revendications

1. Structure de flexion (6) pour un tube d'insertion (2) d'un dispositif médical (1), comportant des vertèbres tubulaires (16) avec une vertèbre tubulaire proximale (16p) et une vertèbre tubulaire distale (16d) et au moins un câble d'actionnement (13) entouré par une gaine (13g) sur au moins une partie de sa longueur, la structure de flexion (6) comportant un tube (15) découpé par des traits de découpe (T) d'un faisceau d'énergie pour réaliser des vertèbres tubulaires (16) imbriquées les unes dans les autres par des zones découpées (17m, 17f, 18m, 18f, 17'm, 17'f, 18'm, 18'f) formant des pivots de rotation selon des axes de pivotement (X, Y), des traits de découpe étant aménagées pour délimiter entre les vertèbres tubulaires (16) des entailles (20, 21, 20', 21') découpées dans le tube pour former des zones de flexion pour les vertèbres tubulaires afin d'obtenir la flexion de la structure de flexion dans au moins un plan de flexion (D, S) perpendiculaire aux axes de pivotement (X, Y), la vertèbre tubulaire proximale (16p) comportant pour chaque câble d'actionnement, un système de blocage axial et radial (40) pour la gaine comportant une découpe (41) allongée aménagée pour délimiter deux bords d'arrêt (41a) limitant l'engagement radial de la gaine à l'intérieur de la découpe, cette découpe débouchant à sa partie distale dans une lumière (43) délimitée par au moins deux lignes de découpe entre lesquelles est attachée une languette d'appui (44) aménagée pour solliciter radialement la gaine en appui contre les deux bords d'arrêt de la découpe, cette lumière (43) étant bordée par un rebord (45a) d'arrêt axial de la gaine sur lequel l'extrémité distale (13d) de la gaine est en appui.

2. Structure de flexion selon la revendication précédente, selon laquelle la vertèbre tubulaire proximale (16p) comporte à sa partie proximale, des languettes de liaison (48) s'étendant axialement et comportant une déformation radiale (48a) pour s'engager dans la partie distale du tube d'insertion (2).

3. Structure de flexion selon la revendication précédente, selon laquelle la vertèbre tubulaire proximale (16p) comporte entre deux languettes voisines (48), des dents (49) comportant des bords d'extrémité (49a) venant en butée sur le bord de l'extrémité distale du tube d'insertion (2).

4. Structure de flexion selon l'une des revendications précédentes, selon laquelle la vertèbre tubulaire distale (16d) est pourvue d'un système de positionnement (30) du câble d'actionnement (13) pour son soudage sur la vertèbre tubulaire distale (16d), ce système de positionnement comportant une languette d'appui (31) découpée du tube par deux lignes de découpe parallèles, en restant attachée au tube à ses extrémités par des zones d'attache et deux contre languettes (33) s'étendant de part et d'autre de la languette d'appui en étant chacune découpée du tube par deux lignes de découpe parallèles, en restant attachée au tube à ses extrémités par des zones d'attache, le câble d'actionnement (13) étant positionné avant soudure entre les contre languettes (33) et la languette d'appui (31), au moins une soudure (13s) permettant de fusionner le câble d'actionnement (13) à la languette d'appui (31).

5. Structure de flexion selon l'une des revendications précédentes, selon laquelle la vertèbre tubulaire distale (16d) est pourvue d'un système d'assemblage (28) avec une tête distale comportant au moins deux découpes d'insertion pour un doigt (4a) de la tête distale (4), aménagées en s'ouvrant à la partie distale par un canal d'engagement (29c) pour le doigt, au moins une découpe présentant deux pointes (29p) de blocage pour un doigt de la tête distale, s'étendant de part et d'autre du canal d'engagement de cette découpe.

6. Structure de flexion selon l'une des revendications précédentes, selon laquelle le tube (15) est découpé par une première série de zones découpées (17m, 17f) formant des pivots de rotation et par une deuxième série de zones découpées (18m, 18f) formant des pivots de rotation s'étendant diamétralement opposée par rapport à la première série pour former des premiers axes de pivotements (X), des traits de découpe étant aménagées pour délimiter entre les vertèbres tubulaires (16), au moins une première série d'entailles (20) pour former des zones de flexion pour les vertèbres tubulaires afin d'obtenir la flexion de la structure de flexion dans un sens de la direction perpendiculaire au premier axe de pivotement (X).

7. Structure de flexion selon la revendication précédente, selon laquelle les zones découpées (17m, 17f, 18m, 18f, 17'm, 17'f, 18'm, 18'f) formant des pivots de rotation coopèrent entre elles par leur épaisseur.

8. Structure de flexion selon l'une des revendications précédentes, selon laquelle des vertèbres tubulaires (16) comportent au moins une languette (25) découpée du tube par deux lignes de découpe parallèles (25a), en restant attachée au tube à ses extrémités par des zones d'attache (25b), la languette (25) étant repoussée à l'intérieur du tube (15) pour constituer un œil de guidage pour le câble d'actionnement (13).

9. Ensemble comportant un tube d'insertion (2) d'un dispositif médical (1) du type endoscope ou cathéter et une structure de flexion (6) conforme à l'une des revendications précédentes, la structure de flexion (6) étant fixée à son extrémité proximale au tube d'insertion (2) et à son extrémité distale, à une tête distale (4).

10. Dispositif médical (1) comportant une poignée de commande (3) équipée à sa partie distale d'un ensemble conforme à la revendication précédente. 1

## Patentansprüche

1. Biegestruktur (6) für ein Einführrohr (2) einer medizinischen Vorrichtung (1), umfassend Rohrwirbel (16) mit einem proximalen Rohrwirbel (16p) und einem distalen Rohrwirbel (16d) und mindestens ein Betätigungskabel (13), das über mindestens einen Teil seiner Länge von einer Hülle (13g) umgeben ist, die Biegestruktur (6) umfassend ein Rohr (15), das durch Schnittlinien (T) eines Energiestrahls geschnitten wird, um rohrförmige Wirbel (16) herzustellen, die durch geschnittene Bereiche (17m, 17f, 18m, 18f, 17'm, 17'f, 18'm, 18'f) ineinander verschachtelt sind, die Drehzapfen für eine Drehung entlang von Schwenkachsen (X, Y) bilden, wobei die Schnittlinien angeordnet sind, um zwischen den rohrförmigen Wirbeln (16) Einschnitte (20, 21, 20', 21') zu begrenzen, die in das Rohr geschnitten sind, um Biegebereiche für die rohrförmigen Wirbel zu bilden, um die Biegung der Biegestruktur in mindestens einer Biegeebene (D, S) senkrecht zu den Schwenkachsen (X, Y) zu erlangen, der proximale rohrförmige Wirbel (16p) für jedes Betätigungskabel ein axiales und radiales Arretiersystem (40) für die Hülle umfasst, umfassend einen länglichen Ausschnitt (41), der ausgebildet ist, um zwei Anschlagkanten (41a) zu begrenzen, die den radialen Eingriff der Hülle in das Innere des Ausschnitts begrenzen, wobei dieser Ausschnitt an seinem distalen Teil in ein Lumen (43) mündet, das von mindestens zwei Ausschnittlinien begrenzt wird, zwischen denen eine Stützlasche (44) angebracht ist, die ausgebildet ist, um die Hülle radial in Anlage gegen die zwei Anschlagkanten des Ausschnitts zu bringen, wobei dieses Lumen (43) von einem axialen Anschlagrand (45a) der Hülle begrenzt wird, auf dem das distale Ende (13d) der Hülle aufliegt.

2. Biegestruktur nach dem vorherigen Anspruch, wobei der proximale rohrförmige Wirbel (16p) an seinem proximalen Teil Verbindungslaschen (48) aufweist, die sich axial erstrecken und eine radiale Verformung (48a) umfassen, um in den distalen Teil des Einführrohrs (2) einzugreifen.

3. Biegestruktur nach dem vorherigen Anspruch, wobei der proximale rohrförmige Wirbel (16p) zwischen zwei benachbarten Laschen (48) Zähne (49) aufweist, die mit Endkanten (49a) umfassen, die an der Kante des distalen Endes des Einführrohrs (2) anliegen.

4. Biegestruktur nach einem der vorherigen Ansprüche, wobei der distale rohrförmige Wirbel (16d) mit einem Positionierungssystem (30) für das Betätigungskabel (13) versehen ist, um es an den distalen rohrförmigen Wirbel (16d) zu schweißen, wobei dieses Positionierungssystem eine Stützlasche (31), die durch zwei parallele Schnittlinien aus dem Rohr ausgeschnitten ist, wobei sie an ihren Enden durch Befestigungszonen an dem Rohr befestigt bleibt, und zwei Gegenlaschen (33) umfasst, die sich auf beiden Seiten der Stützlasche erstrecken, wobei jede durch zwei parallele Schnittlinien aus dem Rohr ausgeschnitten ist, wobei sie an ihren Enden durch Anbringungszonen an dem Rohr befestigt bleibt, das Betätigungskabel (13) vor dem Verschweißen zwischen den Gegenlaschen (33) und der Stützlasche (31) positioniert wird, mindestens eine Lötstelle (13s) es ermöglicht, das Betätigungskabel (13) mit der Stützlasche (31) zu verschmelzen.

5. Biegestruktur nach einem der vorherigen Ansprüche, wobei der distale rohrförmige Wirbel (16d) mit einem Montagesystem (28) mit einem distalen Kopf versehen ist, umfassend mindestens zwei Einführausschnitte für einen Finger (4a) des distalen Kopfs (4), die ausgebildet sind, um sich an dem distalen Teil durch einen Eingriffskanal (29c) für den Finger zu öffnen, wobei mindestens ein Ausschnitt zwei Arretierspitzen (29p) für einen Finger des distalen Kopfs aufweist, die sich auf beiden Seiten des Eingriffskanals dieses Ausschnitts erstrecken.

6. Biegestruktur nach einem der vorherigen Ansprüche, wobei das Rohr (15) durch eine erste Reihe von ausgeschnittenen Bereichen (17m, 17f), die Drehzapfen bilden, und durch eine zweite Reihe von ausgeschnittenen Bereichen (18m, 18f), die Drehzapfen bilden, die sich diametral gegenüber der ersten Reihe erstrecken, um erste Schwenkachsen (X) zu bilden, ausgeschnitten ist, wobei Schnittlinien angeordnet sind, um zwischen den rohrförmigen Wirbeln (16) mindestens eine erste Reihe von Einschnitten (20) zu begrenzen, um Biegebereiche für die rohrförmigen Wirbel zu bilden, um die Biegung der Biegestruktur in einer Richtung der Richtung senkrecht zu der ersten Schwenkachse (X) zu erlangen.

7. Biegestruktur nach dem vorherigen Anspruch, wobei die ausgeschnittenen Bereiche (17m, 17f, 18m, 18f, 17'm, 17'f, 18'm, 18'f), die Drehzapfen bilden, über ihre Stärke miteinander zusammenwirken.

8. Biegestruktur nach einem der vorherigen Ansprüche, wobei rohrförmige Wirbel (16) mindestens eine Lasche (25) umfassen, die durch zwei parallele Schnittlinien (25a) aus dem Rohr ausgeschnitten ist, wobei sie an ihren Enden durch Anbringungsbereiche (25b) an dem Rohr befestigt bleibt, wobei die Lasche (25) in das Innere des Rohrs (15) gedrückt wird, um ein Führungsauge für das Betätigungskabel (13) zu bilden.

9. Anordnung, umfassend ein Einführrohr (2) einer medizinischen Vorrichtung (1) vom Typ Endoskop oder Katheter und einer Biegestruktur (6) nach einem der vorherigen Ansprüche, wobei die Biegestruktur (6) an ihrem proximalen Ende an dem Einführrohr (2) und an ihrem distalen Ende an einem distalen Kopf (4) befestigt ist.

10. Medizinische Vorrichtung (1), umfassend einen Bediengriff (3), der an seinem distalen Teil mit einer Anordnung nach dem vorherigen Anspruch ausgestattet ist.

## Claims

1. A flexing structure (6) for an insertion tube (2) of a medical device (1), comprising tubular vertebrae (16) with a proximal tubular vertebra (16p) and a distal tubular vertebra (16d) and at least one actuating cable (13) surrounded by a sheath (13g) over at least part of its length, the flexing structure (6) comprising a tube (15) cut with energy-beam cutting lines (T) to create tubular vertebrae (16) that nest in one another via cut-out areas (17m, 17f, 18m, 18f, 17'm, 17'f, 18'm, 18'f) forming pivots for rotation around pivot axes (X, Y), cutting lines being created to delimit, between the tubular vertebrae (16), notches (20, 21, 20', 21') cut in the tube in order to form flexing zones for the tubular vertebrae in order to obtain the flexing of the flexing structure in at least one flexing plane (D, S) perpendicular to the pivot axes (X, Y), the proximal tubular vertebra (16p) comprising, for each actuating cable, a radial and axial blocking system (40) for the sheath, comprising an elongate cutout (41) created in such a way as to delimit two stop edges (41a) that limit the radial engagement of the sheath in the cutout, this cutout opening at its distal part into a slot (43) delimited by at least two cutout lines between which there is attached a pressing tab (44) designed to press the sheath radially against the two stop edges of the cutout, this slot (43) being bordered by a rim (45a) for axially stopping the sheath, on which the distal end (13d) of the sheath is pressed.

2. Flexing structure according to the preceding claim, wherein the proximal tubular vertebra (16p) comprises, at its proximal part, connection tabs (48) extending axially and comprising a radial deformation (48a) for engaging in the distal part of the insertion tube (2).

3. Flexing structure according to the preceding claim, wherein the proximal tubular vertebra (16p) comprises two neighbouring tabs (48), teeth (49) comprising end edges (49a) abutting on the distal end edge of the insertion tube (2).

4. Flexing structure according to one of the preceding claims, wherein the distal tubular vertebra (16d) is provided with a positioning system (30) of the actuating cable (13) for its welding on the distal tubular vertebra (16d), this positioning system comprising a pressing tab (31) cut from the tube by two parallel cutout lines, while remaining attached to the tube at its ends by attachment zones and two counter tabs (33) extending on either side of the pressing tab each being cut from the tube by two parallel cutout lines, while remaining attached to the tube at its ends by attachment zones, the actuating cable (13) being positioned before welding between the counter tabs (33) and the pressing tab (31), at least one weld (13s) enabling fusing of the actuating cable (13) to the pressing tab (31).

5. Flexing structure according to one of the preceding claims, wherein the distal tubular vertebra (16d) is provided with an assembly system (28) with a distal head comprising at least two insertion cutouts for a finger (4a) of the distal head (4), created opening at the distal part by an engagement channel (29c) for the finger, at least one cutout having two blocking points (29p) for a finger of the distal head, extending on either side of the engagement channel of this cutout.

6. Flexing structure according to one of the preceding claims, wherein the tube (15) is cut by a first series of cut-out areas (17m, 17f) forming rotation pivots and by a second series of cut-out areas (18m, 18f) forming rotation pivots extending diametrically opposite with respect to the first series in order to form first pivot axes (X), cutting lines being created to delimit, between the tubular vertebrae (16), at least one first series of notches (20) to form flexing zones for the tubular vertebrae in order to obtain the flexing of the flexing structure in one sense of the direction perpendicular to the first pivot axis (X).

7. Flexing structure according to the preceding claim, wherein the cut-out areas (17m, 17f, 18m, 18f, 17'm, 17'f, 18'm, 18'f) forming rotation pivots cooperate with one another via their thickness.

8. Flexing structure according to one of the preceding claims, wherein the tubular vertebrae (16) comprise at least one tab (25) cut from the tube by two parallel cutout lines (25a), while remaining attached to the tube at its ends by attachment zones (25b), the tab (25) being pushed back inside the tube (15) in order to constitute a guide eye for the actuating cable (13).

9. A set comprising an insertion tube (2) of a medical device (1) of the endoscope or catheter type and comprising a flexing structure (6) according to one of the preceding claims, the flexing structure (6) being fixed at its proximal end to the insertion tube and at its distal end to a distal head (4).

10. Medical device (1) comprising a control handle (3) equipped at its distal part with a set according to the preceding claim.
